# EUROPEAN PATENT APPLICATION

(11) **EP 3 653 192 A1**
(43) Date of publication of application: **20.05.2020**
(21) Application number: 19208866.4
(22) Date of filing: 13.11.2019
(51) Int. Cl.: A61H 99/00, A61H 23/02

(54) **MULTIFUNCTIONAL ENTERTAINMENT, PHYSIOTHERAPY AND HEALTH-CARE DEVICE BASED ON CLOUD PLATFORM AND BUS STRUCTURE**

(30) Priority: 15.11.2018 CN 201811360549
(71) Applicant: Guangzhou Pusheng Audio Equipment Co., Ltd., Guangzhou, Guangdong 511448 (CN)
(72) Inventor: CHEN, Hongxian, Guangzhou, Guangdong 511448 (CN)
(74) Representative: de Arpe Fernandez, Manuel

(57) **Abstract**

A multifunctional entertainment, physiotherapy and health-care device based on a cloud platform and a bus structure. The device comprises a cloud platform, a mobile control terminal, a device body, and an industrial control host disposed on the device body, wherein several entertainment and health-care assemblies which are of a bus-type structure and are connected to the industrial control host are also disposed on the device body, and the cloud platform and a control APP on the mobile control terminal control the entertainment and health-care assemblies by means of the industrial control host. In the present invention, the entertainment and health-care assemblies of the plug-and-play bus-type structure are disposed on the device body, and are controlled by means of the control APP on the mobile control terminal, so as to provide people with a variety of entertainment and health-care functions.

## Description

### Technical Field

The present invention relates to the technical field of entertainment, physiotherapy and health-care devices, and in particular to a multifunctional entertainment, physiotherapy and health-care device based on a cloud platform and a bus structure.

### Background Art

At present, there are: many leisure articles, including backrest cane chairs, beach chairs, and sofas; entertainment articles, including computer game machines; and health-care articles, including massage chairs. The physiotherapy device has a low frequency and medium frequency physiotherapy device. These articles are of various kinds, in fashionable styles, and with aesthetic and elegant appearances, and all have entered thousands of households and are popular among people. However, these products each have only one function, for example, a leisure chair can only provide a person with a seat to sit or lie thereon in a leisure way; the massage chair can only provide a person with massages, which relieve the rigidity of muscles, improve physical fitness; and the computer game machine can only provide a function possessed by itself, that is, letting people see the screen, and operate the program keyboard autonomously at most. Therefore, when people see an image on the computer screen, they only have the feeling of watching ordinary video, but not the feeling and pleasure of being present. Therefore, although these products have their respective functions, they cannot allow people to enjoy services of all the above functions at the same time in a certain time, and do not have the functions of physiotherapy to dredge the meridians and relieve pain, so there is a sense of imperfection.

### Summary of the Invention

The present invention provides a multifunctional entertainment, physiotherapy and health-care device based on a cloud platform and a bus structure, wherein entertainment, physiotherapy and health-care assemblies of a plug-and-play bus-type structure are disposed on a device body and are controlled by means of a mobile control terminal, so as to provide people with a variety of entertainment, physiotherapy and health-care functions.

The technical solution of the present invention is implemented as follows:
A multifunctional entertainment, physiotherapy and health-care device based on a cloud platform and a bus structure comprising a cloud platform, a mobile control terminal, a device body, and an industrial control host disposed on the device body, wherein several entertainment, physiotherapy and health-care assemblies which are of a bus-type structure and are connected to the industrial control host are also disposed on the device body, and the cloud platform and a control APP on the mobile control terminal control the entertainment, physiotherapy and health-care assemblies by means of the industrial control host.

Preferably, the entertainment, physiotherapy and health-care assemblies comprise one or more of a near field full frequency speaker with retractable and steering function, a subwoofer, a retractable angle-adjustable projection device, a VR helmet, a lighting scene, an indoor hazardous gas detection device, heating eye patch, a graphene heating physiotherapy electrode sheet, a massage device, a health wristband, an abdominal fat removal machine, a monitoring camera, a face recognition camera, an earphone interface, a code scanning device, a gamepad, an electric wheelchair handle, a vapor mask, an emergency help button, and a prenatal education device.

Preferably, the massage device comprises but is not limited to a massage, graphene and heating pillow, a back movable massage device, a back heating device, a hip massage device, a hip heating device, and a leg massage and heating device.

Preferably, the near field full frequency speaker is fixed to either side of the head of the device body by means of an adjusting rod.

Preferably, the subwoofer is fixed to the bottom, side or rear of the device body to achieve human body resonance.

Preferably, an audio power amplifier is further comprised, wherein the audio power amplifier has a multi-channel power amplifier and is connected to the near field full frequency speaker, the subwoofer and an audio transformer.

Preferably, the massage device further also comprises a music physiotherapy device that amplifies a music signal through the multi-channel power amplifier and then converts the music signal into a current signal through an audio transformer, the current signal being connected to the graphene heating physiotherapy electrode sheet, the graphene heating physiotherapy electrode sheet stimulates the vein and the painful part to reach the effect of dredging the meridians and alleviating the pain.

Preferably, a device corresponding to karaoke is further comprised. Users can freely sing their favorite songs through the device.

Preferably, the lighting scene and heating eye patch comprises an OLED light source and heating eye patch, which is controlled to provide an optimal light scene for sleeping through a bus interface, or provides hot pack for relaxation by means of the heating eye patch.Preferably, the industrial control host comprises several bus-type interfaces and a network communication module, the bus-type interfaces are connected to the entertainment, physiotherapy and health-care device, and the network communication module exchanges data with the cloud platform and the mobile control terminal.

Preferably, a mobile control device is further comprised, wherein the mobile control device interacts data with the industrial control host via the bus-type interfaces or over a wireless network.

Preferably, the industrial control host is further used to control an indoor lighting system and a smart home, and realize the functions of face health recognition, environmental gas monitoring and user health data return in indoors.

The beneficial effects of the present invention are as follows: The entertainment, physiotherapy and health-care assemblies of the plug-and-play bus-type structure are disposed on the device body, and are controlled by the mobile control terminal, so as to provide people with a variety of entertainment, physiotherapy and health-care functions.

### Brief Description of the Drawings

In order to describe the technical solution in embodiments of the present invention or the prior art more clearly, the drawings which need to be used in the description of the embodiments or the prior art will be simply introduced below. Obviously, the accompanying drawings in the following description show merely some embodiments of the present invention, and those of ordinary skill in the art may still derive other drawings according to these drawings without creative efforts.
FIG. 1 is a schematic block diagram of an embodiment of a multifunctional entertainment, physiotherapy and health-care device based on a cloud platform and a bus structure in the present invention;
FIG. 2 is a schematic block diagram of the entertainment, physiotherapy and health-care assemblies; and
FIG. 3 is a schematic structural diagram of the entertainment, physiotherapy and health-care assemblies.

In the figures, 1 - cloud platform; 2 - device body; 3 - industrial control host; 4 - entertainment and health-care assembly; 401 - near field full frequency speaker with retractable and steering function; 402 - subwoofer; 403 - audio power amplifier; 404 - retractable angle-adjustable projection device; 405 - VR helmet; 406 - lighting scene and heating eye patch; 407 - massage, graphene and heating pillow; 408 - back movable massage device; 409 - back heating device; 410 - health wristband; 411 - abdominal fat removal machine; 412 - vapor mask control interface; 413 - prenatal education device interface; 414 - emergency help button; 415 - hip massage device; 416 - hip heating device; 417 - monitoring camera; 418 - earphone interface; 419 - code scanning device; 420 - gamepad; 430 - karaoke device; 431- indoors dangerous gas monitoring device; 432 - face recognition camera; 433 - graphene heating physiotherapy electrode sheet.

### Detailed Description of Embodiments

The technical solution of the embodiments of the present invention will be clearly and completely described below in conjunction with the accompanying drawings in the embodiments of the present invention; and obviously, the embodiments described are merely some of, rather than all, the embodiments of the present invention. On the basis of the embodiments of the present invention, all the other embodiments obtained by those of ordinary skill in the art without creative efforts shall fall within the scope of protection of the present invention.

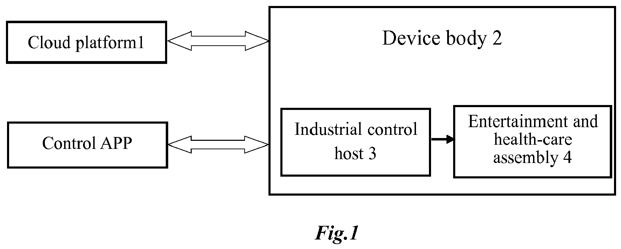

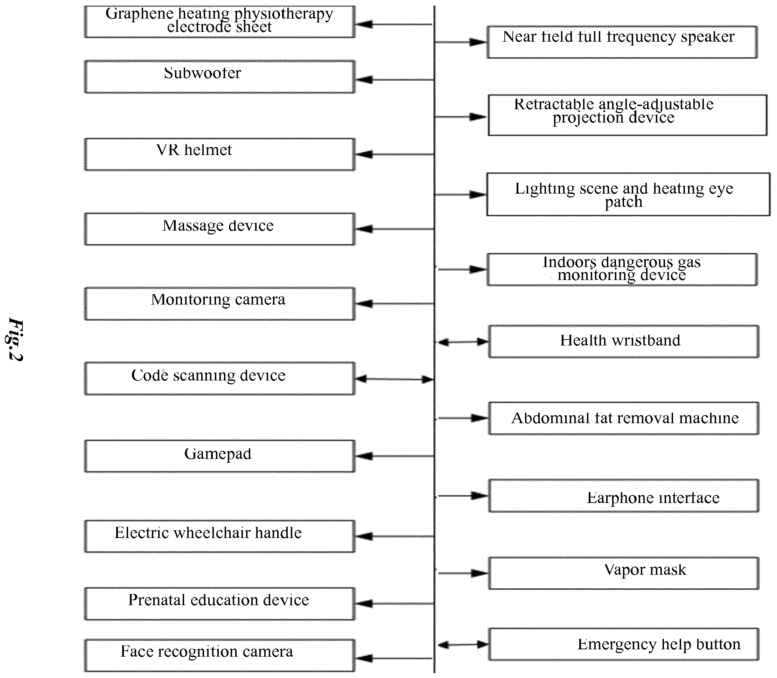

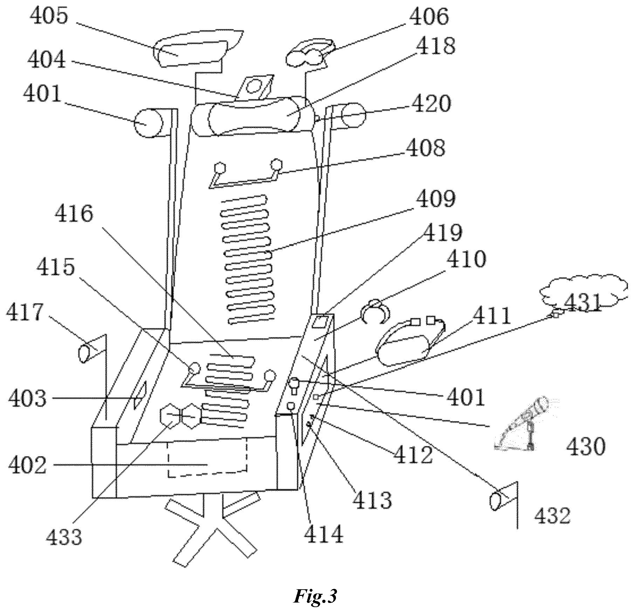

As shown in FIGs. 1 to 3, the present invention provides a multifunctional entertainment, physiotherapy and health-care device based on a cloud platform and a bus structure. The device comprises a cloud platform 1, a mobile control terminal, a device body 2, and an industrial control host 3 disposed on the device body 2, wherein several entertainment, physiotherapy and health-care assemblies 4 which are of a bus-type structure and are connected to the industrial control host 3 are also disposed on the device body 2, and the cloud platform 1 and a control APP on the mobile control terminal control the entertainment and health-care assemblies 4 by means of the industrial control host 3.

Specifically, the present invention provides a multifunctional entertainment, physiotherapy and health-care device based on cloud platform control, wherein the entertainment, physiotherapy and health-care assemblies 4 are of a bus-type structure and are connected to the industrial control host 3, that is, the industrial control host 3 controls the several entertainment, physiotherapy and health-care assemblies 4 via a control line to conveniently increase or decrease the entertainment, physiotherapy and health-care assemblies 4, thereby improving the fun of people's DIY.

The entertainment, physiotherapy and health-care assemblies 4 comprise one or more of a near field full frequency speaker with retractable and steering function 401, a subwoofer 402, a retractable angle-adjustable projection device 404, karaoke device 430, a VR helmet 405, a lighting scene and heating eye patch 406, an indoors dangerous gas monitoring device 431, a massage device, a health wristband 410, an abdominal fat removal machine 411, a monitoring camera 417,a face recognition camera 432, a graphene heating physiotherapy electrode sheet433, an earphone interface 418, a code scanning device 419, a gamepad 420, an electric wheelchair handle, a vapor mask, an emergency help button 414, and a prenatal education device. Specifically, the industrial control host 3 is connected to the above entertainment and health-care assemblies 4 via an RS485 bus.

Specifically, an RS485 interface can be disposed on the device body to facilitate connection to the entertainment, physiotherapy and health-care assemblies 4. The industrial control host 3 can control the working of all the entertainment, physiotherapy and health-care assemblies 4, and can also control the working of certain entertainment, physiotherapy and health-care assemblies 4.

The industrial control host 3 sends a control command via the RS485 bus, and sorting is performed according to the order in which various entertainment, physiotherapy and health-care assemblies 4 receive the command. If the first bit of the control command is 1, it represents that all the entertainment, physiotherapy and health-care assemblies 4 start working, otherwise the first bit is 0. If one or more of the second bit to the N^{th} bit of the control command are 1, it represents that a corresponding entertainment and health-care assembly starts working or corresponding entertainment, physiotherapy and health-care assemblies start working.

The number of entertainment, physiotherapy and health-care assemblies 4 can be set manually or determined by the number of response messages obtained after the industrial control host sends the command.

A vapor mask control interface 412 and a prenatal education device interface 413 are disposed on the device body 2.

The massage device comprises but is not limited to a massage, graphene and heating pillow 407, a back movable massage device 408, a back heating device 409, a hip massage device 415, a hip heating device 416, and a leg massage and heating device. The massage device is controlled by the industrial control host 3 via the bus.

The near field full frequency speaker with retractable and steering function 401 is fixed to either side of the head of the device body 2 by means of an adjusting rod. The movable near-listening speaker 401 can be adjusted up and down according to the height of the human body, and can also be adjusted by 360 degrees in all directions, to provide users with the best listening effect. The adjusting rod can be adjusted manually or electrically. The near field full frequency speaker with retractable and steering function 401 uses a specially made "sandwich" structural composite material with a upper high-frequency limit of up to 20 KHz and a sound pressure of up to 105dB, bring a HI-END level super listening shock to the users.

The subwoofer 402 is fixed to the bottom, side or rear of the device body 2 to achieve human body resonance. The subwoofer uses a subwoofer loudspeaker of a special sound structure and a cavity with a lower low-frequency limit of up to 25 Hz, which can provide super shocking subwoofer energy.

The present invention further comprises an audio power amplifier 403, wherein the speaker power amplifier 403 has a multi-channel power amplification function and is connected to the near field full frequency speaker 401, the subwoofer 402 and an audio transformer. The audio power amplifier 403 can have a multi-channel power amplifier with a network control interface such as RS485, and can be combined with an SRS virtual surround sound module to improve the feeling of watching movies as being present.

The massage device further also comprises a music physiotherapy device that amplifies a music signal through the multi-channel power amplifier and then converts the music signal into a current signal through an audio transformer, the current signal being connected to the graphene heating physiotherapy electrode sheet 433, the graphene heating physiotherapy electrode sheet 433 stimulates the vein and the painful part to reach the effect of dredging the meridians and alleviating the pain.

The earphone interface 418 can be disposed at an output end of the audio power amplifier 403, and the industrial control host 3 controls the switch, the volume, etc. of the earphone interface 418 via the bus.

As the name implies, it can be learned that the projection angle of the retractable angle-adjustable projection device 404 can be adjusted, and specifically, the adjustment can be performed manually or electrically.

The lighting scene and heating eye patch 406 comprises an OLED light source and heating eye patch, which is controlled to provide an optimal light scene mode for sleeping through a bus interface, or provides hot pack for relaxation by means of the heating eye patch.

The indoors dangerous gas monitoring device 431 is used to detect indoor gas leakage, oxygen content, PM2.5 value, etc.

The health wristband 410 is controlled by the industrial control host 3 to collect data such as the blood pressure and heartbeat of a user via the bus, and same can be remotely sent to the cloud platform 1 to determine whether the data such as the blood pressure and heartbeat of the user is normal.

The emergency help button 414 can be used, by pushing the button, to send an emergency help signal to the cloud platform 1 or to trigger a linkage alarm device.

The monitoring camera 417 is connected to the control bus of the industrial control host 3 and can be used for network broadcast or elderly care.

The face recognition camera 432 is used for patient health data comparison, and automatically initiates call and remote video transmission when an abnormality is found.

As for the code scanning device 419, each of the entertainment, physiotherapy and health-care assemblies 4 can be classified into assemblies with free and charging modes, and an entertainment, physiotherapy and health-care assembly 4 with a charging mode is used by means of the code scanning device 419.

The gamepad 420 can be used with the VR helmet 405 for users to play VR games, and the VR helmet 405 can also be used separately with new media video. In addition, the gamepad 420 can also be used with the angle-adjustable projection device 404 to play games.

The electric wheelchair handle provides use of the device for special people.

The industrial control host 3 comprises several bus-type interfaces and a network communication module, wherein the bus-type interfaces are connected to the entertainment and health-care device, and the network communication module exchanges data with the cloud platform 1.

Specifically, the industrial control host 3 can be further used to control an indoor lighting system and a smart home, and realize indoor health monitoring, including the functions of face health recognition, environmental gas monitoring and user health data return in indoors.

The industrial control host 3 is also provided with an intelligent voice control function module, which implements the control of the above entertainment, physiotherapy and health-care assemblies by recognizing a voice control command.

The present invention further comprises a mobile control device, wherein the mobile control device interacts data with the industrial control host 3 via the bus-type interfaces or over a wireless network.

The beneficial effects of the present invention are as follows: The entertainment, physiotherapy and health-care assemblies of the plug-and-play bus-type structure are disposed on the device body 2, and are controlled by means of the cloud platform 1 and the control APP, so as to provide people with a variety of entertainment, physiotherapy and health-care functions.

The above technical solution discloses the improvement of the present invention, and the technical content not disclosed in detail can be implemented by those skilled in the art by means of the prior art.

The above embodiments are merely preferred embodiments of the present invention but not intended to limit the present invention, and any modifications, equivalent replacements, improvements, etc. made within the spirit and principle of the present invention should be included within the scope of protection of the present invention.

## Claims

1. A multifunctional entertainment, physiotherapy and health-care device based on a cloud platform and a bus structure, comprising a cloud platform, a mobile control terminal, a device body, and an industrial control host disposed on the device body, wherein several entertainment, physiotherapy and health-care assemblies which are of a bus-type structure and are connected to the industrial control host are also disposed on the device body, and the cloud platform and a control APP on the mobile control terminal control the entertainment, physiotherapy and health-care assemblies by means of the industrial control host.

2. The multifunctional entertainment, physiotherapy and health-care device based on a cloud platform and a bus structure according to claim 1, wherein the entertainment and health-care assemblies comprise one or more of a near field full frequency speaker with retractable and steering function, a subwoofer, a retractable angle-adjustable projection device, a VR helmet, a lighting scene, an indoor hazardous gas detection device, heating eye patch, a graphene heating physiotherapy electrode sheet, a massage device, a health wristband, an abdominal fat removal machine, a monitoring camera, a face recognition camera, an earphone interface, a code scanning device, a gamepad, an electric wheelchair handle, a vapor mask, an emergency help button, and a prenatal education device.

3. The multifunctional entertainment, physiotherapy and health-care device based on a cloud platform and a bus structure according to claim 2, wherein the massage device comprises, but is not limited to, a massage, graphene and heating pillow, a back movable massage device, a back heating device, a hip massage device, a hip heating device, and a leg massage and heating device.

4. The multifunctional entertainment, physiotherapy and health-care device based on a cloud platform and a bus structure according to claim 2, wherein the near field full frequency speaker is fixed to either side of the head of the device body by means of an adjusting rod.

5. The multifunctional entertainment, physiotherapy and health-care device based on a cloud platform and a bus structure according to claim 2, wherein the subwoofer is fixed to the bottom, side or rear of the device body to achieve human body resonance.

6. The multifunctional entertainment, physiotherapy and health-care device based on a cloud platform and a bus structure according to claims 4 or 5, further comprising an audio power amplifier, wherein the audio power amplifier has a multi-channel power amplifier and is connected to the near field full frequency speaker, the subwoofer and an audio transformer.

7. The multifunctional entertainment and health-care device based on a cloud platform and a bus structure according to claim 6, the massage device further also comprises a music physiotherapy device that amplifies a music signal through the multi-channel power amplifier and then converts the music signal into a current signal through an audio transformer, the current signal being connected to the graphene heating physiotherapy electrode sheet, the graphene heating physiotherapy electrode sheet stimulates the vein and the painful part to reach the effect of dredging the meridians and alleviating the pain.

8. The multifunctional entertainment, physiotherapy and health-care device based on a cloud platform and a bus structure according to claim 2, wherein the lighting scene and heating eye patch comprises an OLED light source and heating eye patch, which is controlled to provide an optimal light scene for sleeping through a bus interface, or provides hot pack for relaxation by means of the heating eye patch.

9. The multifunctional entertainment, physiotherapy and health-care device based on a cloud platform and a bus structure according to claim 1, wherein the industrial control host comprises several bus-type interfaces and a network communication module, the bus-type interfaces are connected to the entertainment, physiotherapy and health-care device, and the network communication module exchanges data with the cloud platform and the mobile control terminal.

10. The multifunctional entertainment, physiotherapy and health-care device based on a cloud platform and a bus structure according to claim 8, further comprising a mobile control device, wherein the mobile control device interacts data with the industrial control host via the bus-type interface or over a wireless network.

11. The multifunctional entertainment, physiotherapy and health-care device based on a cloud platform and a bus structure according to claim 1, wherein the industrial control host is further used to control an indoor lighting system and smart home, and realize the functions of face health recognition, environmental gas monitoring and user health data return in indoors.
